# EUROPÄISCHE PATENTANMELDUNG

(11) **EP 4 190 958 A1**
(43) Veröffentlichungstag der Anmeldung: **07.06.2023**
(21) Anmeldenummer: 22207720.8
(22) Anmeldetag: 16.11.2022
(51) Int. Cl.: D06F 35/00, D06F 39/00, D06F 33/43, D06F 37/26, D06F 103/68, D06F 105/00, D06F 105/38

(54) **VERDAMPFUNGSVORRICHTUNG FÜR EIN WÄSCHEPFLEGEGERÄT, WÄSCHEPFLEGEGERÄT UND VERFAHREN ZUM VERDAMPFEN EINER FLÜSSIGKEIT IN EINEM WÄSCHEPFLEGEGERÄT**

(30) Priorität: 01.12.2021 DE 102021213574
(71) Anmelder: BSH Hausgeräte GmbH, 81739 München (DE)
(72) Erfinder: Albayrak, Hasan Gökcer, 14476 Potsdam (DE); Skrippek, Jörg, 14641 Wustermark (DE); Steffens, Günter, 14612 Falkensee (DE); Genc, Kemal, 12347 Berlin (DE)

(57) **Zusammenfassung**

Die Erfindung betrifft eine Verdampfungsvorrichtung (1) für ein Wäschepflegegerät (24), umfassend einen mit einer Flüssigkeit befüllten und/oder befüllbaren Flüssigkeitstank (2), einen Verdampfer (4), der dazu ausgestaltet ist, die Flüssigkeit zu verdampfen, einen Sammelbehälter (6), der dazu ausgestaltet ist, die verdampfte Flüssigkeit aufzunehmen, und einen Pumpenmechanismus (8), der dazu ausgestaltet ist, die verdampfte Flüssigkeit aus dem Sammelbehälter (6) einem Behandlungsraum (12) des Wäschepflegegeräts (24) zuzuführen.

## Beschreibung

Die vorliegende Erfindung betrifft eine Verdampfungsvorrichtung für ein Wäschepflegegerät, ein Wäschepflegegerät und ein Verfahren zum Verdampfen einer Flüssigkeit in einem Wäschepflegegerät.

Zur hygienischen Behandlung von Wäsche wird im Stand der Technik oftmals auf eine Ozonbehandlung der Wäsche zurückgegriffen, bei der das Ozon eine keimabtötende, desinfizierende und dadurch in der Regel auch geruchsmindernde Wirkung entfaltet. Vorteilhafterweise kann dabei auf ein Befeuchten der Wäsche und damit auch auf ein späteres Trocknen der Wäsche verzichtet werden. Diese Behandlung kann jedoch, wie auch andere Wäschebehandlungsprozesse wie beispielsweise ein Trocknen der Wäsche mit Heißluft oder eine Behandlung der Wäsche mit heißem Dampf, den Nachteil haben, dass ein Nutzer beim Blick in einen Behandlungsraum des Wäschepflegegeräts bzw. in die Wäschetrommel, keine visuellen Hinweise oder ein Feedback dafür erhält, dass das Wäschepflegeprogramm durchgeführt wird. Mit anderen Worten kann der Nutzer oft nur schwer erkennen, dass ein Behandlungsprogramm abläuft. Dies ist dadurch bedingt, dass beispielsweise eingeleitetes Ozon oder eingeleitete Heißluft im Wesentlichen unsichtbar für das menschliche Auge ist. Dies kann zu einer Verunsicherung des Nutzers bezüglich der Funktionsfähigkeit des Wäschepflegeprogramms führen. Es hat sich herausgestellt, dass diese Verunsicherung oft zu einer Fehlbedienung des Wäschepflegegeräts durch den Nutzer führt. Ferner kann diese Verunsicherung zu vermehrten Serviceanfragen führen.

Im Stand der Technik wird diesem Problem damit begegnet, dass eine Bedienanzeige einen Prozessstart anzeigt. Gerade bei erstmaligem Einsatz eines Wäschepflegegeräts ist das Vertrauen eines Nutzers in eine solche Bedienanzeige jedoch oftmals eingeschränkt.

Es ist daher eine Aufgabe der Erfindung, eine Möglichkeit bereitzustellen, eine Fehlbedienung eines Wäschepflegegeräts durch einen Nutzer und eine Verunsicherung eines Nutzers zu vermeiden.

Diese Aufgabe wird gelöst durch eine Verdampfungsvorrichtung mit den Merkmalen des Anspruchs 1, ein Wäschepflegegerät mit den Merkmalen des Anspruchs 7 und ein Verfahren mit den Merkmalen des Anspruchs 10. Weitere Vorteile und Merkmale der Erfindung ergeben sich aus den Unteransprüchen sowie der Beschreibung und den beigefügten Figuren.

Gemäß einem ersten Aspekt der vorliegenden Erfindung ist eine Verdampfungsvorrichtung für ein Wäschepflegegerät vorgesehen. Die Verdampfungsvorrichtung umfasst einen mit einer Flüssigkeit befüllten und/oder befüllbaren Flüssigkeitstank, einen Verdampfer, der dazu ausgestaltet ist, die Flüssigkeit zu verdampfen, einen Sammelbehälter, der dazu ausgestaltet ist, die verdampfte Flüssigkeit aufzunehmen, und einen Pumpenmechanismus, der dazu ausgestaltet ist, die verdampfte Flüssigkeit aus dem Sammelbehälter einem Behandlungsraum des Wäschepflegegeräts zuzuführen.

Die Verdampfungsvorrichtung kann insbesondere dazu ausgebildet sein, in das Wäschepflegegerät eingebaut zu werden und/oder in dem Wäschepflegegerät integriert zu sein. Der Behandlungsraum kann eine Wäschetrommel des Wäschepflegegeräts umfassen. Die Verdampfungsvorrichtung kann dazu ausgestaltet sein, mit einer Steuervorrichtung des Wäschepflegegeräts zu kommunizieren. Die Steuervorrichtung kann dazu ausgestaltetet sein, Informationen aufzunehmen, zu verarbeiten und Steuerungsbefehle auszugeben. Somit kann die Steuervorrichtung die Verdampfungsvorrichtung steuern. Beispielsweise kann die Verdampfungsvorrichtung dazu ausgestaltet sein, ihren Betrieb aufgrund eines Steuerungsbefehls, insbesondere von der Steuervorrichtung des Wäschepflegegeräts und/oder ausgelöst beim Start eines Wäschepflegeprogramms des Wäschepflegegeräts, zu starten. Vorteilhafterweise kann es die Verdampfungsvorrichtung ermöglichen, durch die verdampfte Flüssigkeit einen visuellen für den Nutzer erfassbaren Hinweis bereitzustellen. Mit anderen Worten kann die verdampfte Flüssigkeit, sobald sie durch den Pumpenmechanismus in den Behandlungsraum geführt wird, einem Nutzer ein direktes visuelles Erkennen eines gestarteten Behandlungsprogramms ermöglichen. Somit kann einem Nutzer ein Feedback über den Ablauf eines Behandlungsprogramms bereitgestellt werden, wenn das Behandlungsprogramm selbst, beispielsweise während eines Zuführens von Ozon in den Behandlungsraum oder bei einem Luftumwälzen während eines Wäschetrocknerprogramms, nicht direkt erkennbar ist.

Der Verdampfer kann dazu ausgestaltet sein, die Flüssigkeit derart zu verdampfen, dass eine verdampfte Flüssigkeit entsteht. Die verdampfte Flüssigkeit kann ein Gas oder ein Gas-Flüssigkeit-Gemisch sein. Die verdampfte Flüssigkeit kann nebelförmig, gasförmig und/oder dampfförmig sein. Optional kann die Verdampfungsvorrichtung, insbesondere der Verdampfer und/oder der Flüssigkeitstank, derart ausgestaltet sein, dass die Flüssigkeit sich beim und/oder nach dem Verdampfen mit einem Fluid aus der Umgebung, insbesondere mit zugeführtem Wasser und/oder mit Luft vermischt, insbesondere zu einem Nebel vermischt. Der Verdampfer kann dazu ausgestaltet sein, die Flüssigkeit zu erwärmen und dadurch zu verdampfen. Vorzugsweise kann der Verdampfer dazu ausgestaltet sein, die Flüssigkeit durch direkten Kontakt und/oder durch Strahlung, insbesondere Wärmestrahlung, zu erwärmen und dadurch zu verdampfen. Der Verdampfer kann in dem Flüssigkeitstank angeordnet sein. Vorteilhafterweise kann dadurch eine besonders direkte und somit effiziente Wärmeübertragung von dem Verdampfer zu der Flüssigkeit ermöglicht sein. Alternativ kann der Verdampfer außerhalb des Flüssigkeitstanks angeordnet sein. Dies kann einen besonders effizienten Austausch oder eine besonders einfache Wartung des Flüssigkeitstanks und/oder des Verdampfers ermöglichen, da beide Komponenten, nämlich der Flüssigkeitstank und der Verdampfer, unabhängig voneinander ausgetauscht werden können. Der Verdampfer kann so angeordnet sein, dass ein Bereich des Flüssigkeitstanks und/oder die sich in diesem Bereich befindliche Flüssigkeit durch den Verdampfer erwärmbar ist. Der Verdampfer kann dazu ausgebildet sein, Ultraschallwellen zu erzeugen und damit einen Teil der Flüssigkeit derart zu Schwingungen anzuregen, dass durch Tröpfchenbildung und/oder Zerstäubung Nebel umfassend Tröpfchen der Flüssigkeit entsteht und/oder die Flüssigkeit verdampft. Die Flüssigkeit kann insbesondere ein Nebelfluid sein, beispielsweise ein Gemisch umfassend destilliertes Wasser und Propylenglykol. Die Flüssigkeit und/oder die verdampfte Flüssigkeit ist vorzugsweise für das menschliche Auge sichtbar. Sichtbar kann in diesem Zusammenhang bedeuten, dass die verdampfte Flüssigkeit bei direktem Blickkontakt mit dem menschlichen Auge detektierbar bzw. wahrnehmbar ist. Die verdampfte Flüssigkeit kann eine Farbe, die im für das menschliche Auge sichtbaren Frequenzbereich angeordnet ist, aufweisen. Vorzugsweise kann die Flüssigkeit bzw. die verdampfte Flüssigkeit derart ausgebildet sein, dass sie nicht mit Wäsche, Wäschebehandlungsmittel, insbesondere Ozon, und/oder Komponenten des Wäschepflegegeräts reagiert. Mit anderen Worten kann die verdampfte Flüssigkeit vorzugsweise neutral gegenüber der Wäsche, dem Wäschebehandlungsmittel und/oder den Komponenten des Wäschepflegegeräts sein. Die Flüssigkeit kann beispielsweise eine, insbesondere gegenüber Wäsche neutrale, Emulsion sein. Vorteilhafterweise kann mit einer neutralen Flüssigkeit eine Beeinträchtigung der Wäsche und/oder des Behandlungsprogramms vermieden werden. Die Flüssigkeit bzw. die verdampfte Flüssigkeit kann geruchlos sein. Die Flüssigkeit kann eine chemisch inerte Flüssigkeit sein. Somit kann die Flüssigkeit eine Substanz sein, die unter den jeweilig gegebenen Bedingungen mit potentiellen Reaktionspartnern (etwa Luft, Wasser, Edukte und Produkte einer Reaktion) nicht oder nur in verschwindend geringem Maße reagiert. Alternativ kann die Flüssigkeit bzw. die verdampfte Flüssigkeit einen Geruchsstoff enthalten, insbesondere parfümiert sein. Vorteilhafterweise kann somit eine zusätzliche Geruchsbehandlung der Wäsche ermöglicht werden.

Der Flüssigkeitstank kann abnehmbar an der Verdampfungsvorrichtung angeordnet sein. Somit kann der Flüssigkeitstank austauschbar sein. Beispielsweise kann es vorgesehen sein, den Flüssigkeitstank auszutauschen, wenn die Flüssigkeit aufgebraucht ist. Der Flüssigkeitstank kann insbesondere eine austauschbare und/oder nachfüllbare Flüssigkeitspatrone sein. Der Flüssigkeitstank kann eine Öffnung aufweisen, mit welcher der Flüssigkeitstank mit einer Flüssigkeit befüllt werden kann. Somit kann der Flüssigkeitstank nach einem Aufbrauchen der Flüssigkeit nachgefüllt werden. Der Flüssigkeitstank kann ein Fassungsvermögen von 10 ml bis 500 ml, bevorzugt 50 ml bis 300 ml, besonders bevorzugt 100 ml bis 150 ml aufweisen. Ein Fassungsvermögen von 10 ml bis 500 ml kann eine für einen längeren Betrieb günstige Menge an Flüssigkeit bereitstellen. Ein solches Fassungsvermögen kann, unter der Berücksichtigung, dass das Volumen eines Stoffes oder Stoffgemischs beim Übergang vom flüssigen zu einem gasförmigen Zustand sein Volumen deutlich vergrößert, eine für einen langen Gebrauch im Sinne der Erfindung ausreichende Menge an Flüssigkeit bereitstellen und gleichzeitig durch seine Größe gut in ein Wäschepflegegerät integrierbar sein. Besonders vorteilhaft kann dabei ein Fassungsvermögen von 50 ml bis 300 ml sein, insbesondere wenn eine Verwendung der Verdampfungsvorrichtung nur am Anfang eines Wäschepflegeprogramms vorgesehen ist. Dabei kann ein Bereich von 100 ml bis 150 ml insbesondere für die gesamte Lebensdauer eines Wäschepflegegeräts ausreichend sein, wenn eine vorbestimmte begrenzte Nutzungsrate, z.B. einmal wöchentlich, vorgesehen ist.

Zudem kann der Flüssigkeitstank ein Fassungsvermögen aufweisen, welches ausreicht, um die Verdampfungsvorrichtung während einer gesamten Lebensdauer des Wäschepflegegeräts und zu versorgen. Somit kann der Flüssigkeitstank integral mit der Verdampfungsvorrichtung ausgebildet sein. Mit anderen Worten muss die Flüssigkeit nicht nachgefüllt werden oder der Flüssigkeitstank ausgetauscht werden. Dies ermöglicht eine besonders einfache Bedienung des Wäschepflegegeräts.

Der Sammelbehälter kann direkt oder indirekt beispielsweise über ein Verbindungselement, an den Flüssigkeitstank angeschlossen oder anschließbar sein. Vorzugsweise ist der Sammelbehälter an den Bereich des Flüssigkeitstanks angeschlossen, in dem durch den Verdampfer die verdampfte Flüssigkeit erzeugt wird. Der Sammelbehälter kann einen Eingangsabschnitt zur direkten oder indirekten Verbindung mit dem Flüssigkeitstank und/oder einen Ausgangsabschnitt zur direkten oder indirekten Verbindung mit dem Behandlungsraum umfassen. Eine indirekte Verbindung kann beispielsweise über einen Zufuhrschlauch bzw. eine Zuleitung vorgesehen sein. In dem Sammelbehälter kann verdampfte Flüssigkeit gespeichert werten und bei bedarf dem Behandlungsraum zugeführt werden. Dies bietet insbesondere den Vorteil, dass der Verdampfer nicht auf einmal die erforderliche Menge an verdampfter Flüssigkeit erzeugen muss, sondern die erforderliche Menge an verdampfter Flüssigkeit direkt durch den Sammelbehälter bereitgestellt werden kann. Damit kann der Verdampfer mit einer geringeren Leistung realisiert sein. Der Sammelbehälter kann ein geringeres Volumen als der Flüssigkeitstank aufweisen. Somit kann die Verdampfungsvorrichtung besonders kompakt sein.

Der Pumpenmechanismus kann in oder an dem Sammelbehälter angeordnet sein. Alternativ kann der Pumpenmechanismus z.B. in oder an einer an den Ausgangsabschnitt anschließenden Leitung, z.B. dem Zufuhrschlauch, angeordnet sein. Der Pumpenmechanismus kann zusätzlich dazu ausgestaltet sein, die verdampfte Flüssigkeit aus dem Flüssigkeitstank in den Sammelbehälter zu befördern. Ferner kann der Pumpenmechanismus dazu ausgestaltet sein, die verdampfte Flüssigkeit von dem Sammelbehälter zu dem Behandlungsraum zu transportieren. Somit kann der Pumpenmechanismus verdampfte Flüssigkeit von dem Verdampfer ansaugen und von dem Sammelbehälter in den Behandlungsraum befördern, insbesondere blasen.

Gemäß einer bevorzugten Ausführungsform ist vorgesehen, die optische Wahrnehmung der Verdampfung zusätzlich mit einer Trommelbeleuchtung zu koppeln und damit zu verstärken. Mit anderen Worten kann die Verdampfungsvorrichtung eine Beleuchtungseinrichtung umfassen, welche dazu ausgestaltet ist, den Behandlungsraum zumindest teilweise zu beleuchten. Vorzugsweise kann eine Steuereinheit vorgesehen sein, die dazu ausgestaltet ist, die Beleuchtung des Behandlungsraum gleichzeitig mit Zuführen der verdampften Flüssigkeit in den Behandlungsraum zu aktivieren.

Gemäß einer bevorzugten Ausführungsform kann der Pumpenmechanismus einen Blasebalg umfassen, wobei der Pumpenmechanismus zum Antreiben des Blasebalgs insbesondere ein Schneckengetriebe umfassen kann. Ferner kann Pumpenmechanismus ein Antriebsrad mit exzentrischer Drehachse umfassen. Der Blasebalg kann an den Sammelbehälter angeschlossen sein und/oder mit dem Sammelbehälter in Fluidverbindung stehen. Beispielsweise kann der Blasebalg eine, insbesondere untere, Öffnung aufweisen, die an eine, insbesondere obere, Öffnung des Sammelbehälters angeschlossen ist. Die Begriffe unten und oben können sich auf eine Betriebsstellung des Verdampfers beziehen. Der Blasebalg kann beispielsweise aus einem Kunststoffmaterial oder einem Gummimaterial gefertigt sein. Das Antriebsrad kann derart an dem Blasebalg befestigt sein, dass durch seine exzentrische Drehachse ein Drehen des Antriebsrads ein Auf- und Zuziehen des Blasebalgs verursacht. Beispielsweise kann der Blasebalg an einem Umfangsbereich des Antriebsrades befestigt sein. Das Schneckengetriebe kann vorzugsweise dazu ausgestaltet sein, das Antriebsrad anzutreiben. Insbesondere kann das Antriebsrad ein Zahnradgetriebe umfassen, welches durch ein Schraubgetriebe des Schneckengetriebes angetrieben wird. Das Auf- und Zuziehen des an den Sammelbehälter fluidisch angeschlossenen Blasebalgs kann zu einem, insbesondere periodischen bzw. zyklischen, Erzeugen von Über- und Unterdruck in dem Sammelbehälter ausgelegt sein. Der Blasebalg bietet den Vorteil, dass sowohl ein Unterdruck (d.h. ein Ansaugen der verdampften Flüssigkeit weg von dem Verdampfer) als auch ein Überdruck (d.h. ein Ausstoßen der verdampften Flüssigkeit aus dem Sammelbehälter zu dem Behandlungsraum) mit nur einer Vorrichtung möglich ist. Die Verwendung eines Blasebalgs ist zudem eine platzsparende Möglichkeit, die verdampfte Flüssigkeit aus von dem Verdampfer in den Sammelbehälter und von dort in den Behandlungsraum zu transportieren.

Gemäß einer weiteren bevorzugten Ausführungsform der vorliegenden Erfindung ist es vorgesehen, dass der Pumpenmechanismus dazu ausgestaltet ist, in einer Ansaugphase durch Erzeugen von Unterdruck verdampfte Flüssigkeit aus dem Flüssigkeitstank zu saugen, insbesondere in den Sammelbehälter, und in einer Pumpphase durch Erzeugen von Überdruck die verdampfte Flüssigkeit, insbesondere aus dem Sammelbehälter heraus, dem Behandlungsraum zuzuleiten. Der Unterdruck und der Überdruck können durch den Blasebalg erzeugt werden. Mit anderen Worten kann der Pumpenmechanismus dazu ausgestaltet sein, zyklisch abwechselnd dem Sammelbehälter, insbesondere über einen Eingangsabschnitt des Sammelbehälters, verdampfte Flüssigkeit aus dem Flüssigkeitstank zuzuführen und die verdampfte Flüssigkeit, insbesondere über einen Ausgangsabschnitt des Sammelbehälters, aus dem Sammelbehälter hinaus, insbesondere in einen Behandlungsraum, zu leiten. Dieses Erzeugen von Unterdruck und Überdruck kann eine besonders energieeffiziente und gleichzeitig zuverlässige Möglichkeit der Beförderung der verdampften Flüssigkeit darstellen.

Gemäß einer bevorzugten Ausführungsform ist es vorgesehen, dass in einer Verbindung zwischen dem Sammelbehälter und dem Verdampfer ein Eingangsventil angeordnet ist, das dazu ausgestaltet ist, während der Ansaugphase offen und während der Pumpphase geschlossen zu sein und/oder dass in einer Verbindung zwischen dem Sammelbehälter und dem Behandlungsraum ein Ausgangsventil angeordnet ist, das dazu ausgestaltet ist, während der Pumpphase offen und während der Ansaugphase geschlossen zu sein. Das Eingangsventil und/oder das Ausgangsventil kann/können beispielsweise mit dem Antrieb des Pumpmechanismus, insbesondere mechanisch oder elektronisch, gekoppelt sein. Beispielsweise kann der Pumpmechanismus eine Steuereinheit umfassen, welche sowohl die Ansaugphase und die Pumpphase als auch das Öffnen und Schließen des Eingangsventils und/oder des Ausgangsventils steuert. Vorteilhafterweise kann durch das Eingangsventil ein Rückfluss der verdampften Flüssigkeit zu dem Verdampfer verhindert sein. Zudem kann das Eingangsventil dazu ausgestaltet sein, ein Austreten von Flüssigkeit aus dem Flüssigkeitstank, insbesondere bei Nichtbetrieb der Verdampfungsvorrichtung zu verhindern. Das Ausgangsventil kann vorteilhafterweise ein Ansaugen von Gas oder Flüssigkeit aus dem Behandlungsraum und/oder aus der Umgebung in den Sammelbehälter während der Ansaugphase verhindern. Mit anderen Worten können die Ventile einen besonders effizienten und verlustarmen Betrieb der Verdampfungsvorrichtung ermöglichen. Beispielsweise kann das Eingangsventil und/oder das Ausgangsventil als Rückschlagventile ausgestaltet sein. Insbesondere sind das Eingangsventil und das Ausgangsventil mit voneinander verschiedenen Funktionsrichtungen vorgesehen. Somit kann zuverlässig ein Fehlbetrieb der Verdampfungsvorrichtung verhindert werden und ein einfaches System bereitgestellt werden.

Gemäß einer weiteren bevorzugten Ausführungsform ist es vorgesehen, dass die Flüssigkeit in dem Flüssigkeitstank ein Desinfektionsmittel umfasst und/oder dass die Verdampfungsvorrichtung einen Desinfektionsmittelbehälter und eine Desinfektionsmittelzuführleitung umfasst, wobei die Verdampfungsvorrichtung dazu ausgestaltet ist, über die Desinfektionsmittelzuführleitung der verdampften Flüssigkeit Desinfektionsmittel zuzuführen. Insbesondere weil Ozon reaktiv mit z.B. der Wäsche wirken kann, ist eine Menge an zuführbarem Ozon begrenzt. Hier kann das Desinfektionsmittel vorteilhafterweise eine zusätzliche Reinigung bzw. Desinfektion der Wäsche und somit eine Verringerung der zu verwendenden Ozonmenge ermöglichen. Das Desinfektionsmittel in dem Flüssigkeitstank kann zweckmäßigerweise einen ähnlichen Siedepunkt wie die übrige zu verdampfende Flüssigkeit aufweisen, insbesondere einen um nicht mehr als 50°C unterschiedlichen Siedepunkt. Vorteilhafterweise kann somit das Desinfektionsmittel gemeinsam und bei gleicher Temperatur mit der übrigen Flüssigkeit verdampft werden. Alternativ kann das Desinfektionsmittel einen höheren Siedepunkt als die übrige Flüssigkeit aufweisen, wobei der Verdampfer insbesondere dazu ausgestaltet sein kann, dass beim Verdampfen der Flüssigkeit, das Desinfektionsmittel im Wesentlichen in flüssiger Form verbleibt, sodass beim Verdampfen der Flüssigkeit ein Gas-Flüssigkeit-Gemisch entsteht, bei dem das Desinfektionsmittel noch in flüssigem Zustand vorliegt. Analog kann die Verdampfungsvorrichtung dazu ausgestaltet sein, der verdampften Flüssigkeit derart das Desinfektionsmittel zuzuführen, dass das Desinfektionsmittel mit der verdampften Flüssigkeit zu einem Gas-Flüssigkeit-Gemisch vermengt wird. Ein Zuführen des Desinfektionsmittels über die Desinfektionsmittelzuführleitung kann beispielsweise von einer Desinfektionsmittelsteuereinheit der Verdampfungsvorrichtung kontrolliert werden, z.B. über Zusatzventile und/oder einen Desinfektionsmittelfördermechanismus, insbesondere in Form einer Desinfektionsmittelförderpumpe. Die Desinfektionsmittelsteuereinheit kann optional in die Steuereinheit des Pumpmechanismus integriert sein oder mit dieser verbunden sein.

Gemäß einer weiteren bevorzugten Ausführungsform ist es vorgesehen, dass der Verdampfer ein, insbesondere elektrisch betriebenes, Heizelement umfasst. Das Heizelement kann beispielsweise ein Heizstab oder eine Heizspirale sein. Das Heizelement kann durch eine Steuereinheit, beispielsweise die Steuereinheit der Verdampfungsvorrichtung und/oder durch eine externe Steuereinheit regulierbar sein. Eine externe Steuereinheit kann in diesem Sinne beispielsweise eine Steuervorrichtung des Wäschepflegegeräts sein. Das Heizelement kann dazu ausgestaltet sein, die Flüssigkeit in dem Flüssigkeitstank durch direkten Kontakt und/oder über ein Kontaktelement zu erwärmen und dadurch lokal zu verdampfen. Das Heizelement kann eine direkte und damit besonders effiziente Wärmeübertragung an die Flüssigkeit ermöglichen. Zudem kann ein elektrisch betriebenes Heizelement besonders einfach steuerbar sein.

Gemäß einem weiteren Aspekt der vorliegenden Erfindung wird ein Wäschepflegegerät bereitgestellt. Das Wäschepflegegerät umfasst eine Verdampfungsvorrichtung zum Verdampfen von Flüssigkeit, insbesondere eine erfindungsgemäße Verdampfungsvorrichtung wie hierin beschrieben, einen Behandlungsraum, insbesondere mit einer Fluidzufuhr, die dazu ausgestaltet ist, dem Behandlungsraum ein Fluid zuzuführen, wobei der Behandlungsraum mit der Verdampfungsvorrichtung über eine Zuleitung in Kommunikation steht, eine Steuereinheit, die dazu ausgestaltet ist, dem Behandlungsraum, insbesondere während einer Ozonbehandlung und/oder einer Heißluftbehandlung, verdampfte Flüssigkeit zuzuführen. Die Merkmale und Vorteile, die für die Verdampfungsvorrichtung beschrieben worden sind, gelten in entsprechender Weise analog für das Wäschepflegegerät und andersrum. Die verdampfte Flüssigkeit ist insbesondere eine von der Verdampfungsvorrichtung verdampfte Flüssigkeit. Die Steuereinheit kann vorzugsweise dazu ausgestaltet sein, die verdampfte Flüssigkeit mittels der Zuleitung, über die der Behandlungsraum mit der Verdampfungsvorrichtung in Kommunikation steht, in den Behandlungsraum zuzuführen. Die Steuervorrichtung kann dazu ausgestaltet sein mit der Verdampfungsvorrichtung zu kommunizieren. Beispielsweise kann die Steuereinheit dazu ausgestaltet sein, ein Trigger-Signal, insbesondere beim Start oder kurz vor dem Start des Wäschepflegeprogramms des Wäschepflegegeräts, an die Verdampfungsvorrichtung zu senden, um damit der Verdampfungsvorrichtung einen Befehl zum Starten ihres Betriebs zu übermitteln. Die Zuleitung kann beispielsweise ein Verbindungsschlauch sein.

Die Fluidzufuhr kann dazu ausgestaltet sein, dem Behandlungsraum ein Behandlungsfluid, z.B. Wasser, insbesondere mit Waschmittel vermischtes Wasser, Heißluft und/oder Ozon, zuzuführen. Optional kann die Zuleitung an der Fluidzufuhr angeschlossen sein.

Dadurch kann eine besonders einfache Ausführung mit wenigen Zuleitungsöffnungen möglich sein. Alternativ kann die Zuleitung separat von der Fluidzufuhr ausgebildet sein. Eine separate Zuleitung kann vorteilhaft sein, indem dadurch eine bessere Umsetzbarkeit bzw. Kombinierbarkeit von verschiedenen Varianten von Verdampfungsvorrichtungen und/oder Wäschepflegegeräten möglich sein kann. Beispielsweise kann die Zuleitung an Mitnehmern in dem Behandlungsraum, die dem Mitnehmen bzw. Umwälzen der Wäsche dienen, oder an einem den Behandlungsraum umgebenden Laugenbehälter angeordnet sein. Die Steuereinheit kann vorzugsweise dazu ausgestaltet sein, dem Behandlungsraum während eines Wäschebehandlungsprogramms, vorzugsweise während eines Wäschebehandlungsprogramms, bei dem ansonsten dem Behandlungsraum kein für einen Nutzer sichtbares Wäschebehandlungsmittel und/oder Behandlungsfluid zugeführt wird, insbesondere während der Ozonbehandlung und/oder der Heißluftbehandlung, verdampfte Flüssigkeit zuzuführen. Die Steuereinheit kann besonders bevorzugt dazu ausgestaltet sein, dem Behandlungsraum die verdampfte Flüssigkeit zu Beginn bzw. in einer Anfangsphase des Wäschebehandlungsprogramms, insbesondere der Ozonbehandlung und/oder der Heißluftbehandlung, zuzuführen. "Zu Beginn" bzw. in der Anfangsphase kann dabei die ersten 10 bis 120 Sekunden, bevorzugt die ersten 20 bis 60 Sekunden, des Programms bezeichnen. Die Steuereinheit kann dazu ausgestaltet sein, dem Behandlungsraum nach dem Beginn des Wäschebehandlungsprogramms bzw. nach der Anfangsphase keine weitere verdampfte Flüssigkeit zuzuführen. Vorteilhafterweise kann damit die Menge an verbrauchter Flüssigkeit eingeschränkt werden, bzw. es kann Flüssigkeit eingespart werden. Es hat sich gezeigt, dass Nutzer in der Regel den Programmfortschritt nur zu Beginn des Programms beobachten und im weiteren Verlauf das Wäschebehandlungsprogramms weniger genau beobachten. Zudem kann die verdampfte Flüssigkeit nach dem Zuführen ggf. einige Zeit in dem Behandlungsraum verbleiben. Vorteilhafterweise kann somit ein Zuführen zu Beginn des Wäschebehandlungsprogramms ausreichend sein, um den gewünschten Effekt zu erzielen.

Die Verdampfungsvorrichtung kann in einer Betriebsstellung des Wäschepflegegeräts oberhalb des Behandlungsraums angeordnet sein. Beispielsweise kann die Verdampfungsvorrichtung mittig über dem Behandlungsraum angeordnet sein. Besonders bevorzugt kann die Verdampfungsvorrichtung direkt unterhalb einer Arbeitsplatte des Wäschepflegegeräts bzw. unterhalb einer Deckelplatte des Wäschepflegegeräts angeordnet sein. Die Verdampfungsvorrichtung kann eine Schnittstelle nach außen, insbesondere durch die Arbeitsplatte oder durch eine Seitenwand des Wäschepflegegeräts aufweisen. Die Schnittstelle kann dazu ausgestaltet sein, einen Austausch des Flüssigkeitstanks und/oder eine Wartung der Verdampfungsvorrichtung zu ermöglichen.

Gemäß einer bevorzugten Ausführungsform ist es vorgesehen, dass das Wäschepflegegerät eine mit einer Tür verschließbare Öffnung aufweist, wobei an der Öffnung insbesondere eine Dichtmanschette angeordnet ist, wobei die Zuleitung an der Dichtmanschette und/oder in einem Bereich der Tür an dem Behandlungsraum angeschlossen ist. Die Tür kann vorzugsweise ein Sichtfenster umfassen, welches dem Nutzer einen Blick auf den Behandlungsraum bzw. auf die Wäsche in dem Behandlungsraum ermöglicht. Das Sichtfenster kann vorzugsweise eine kreisrunde oder ovale Form aufweisen. Besonders bevorzugt kann die Zuleitung in dem Bereich des Sichtfensters, insbesondere oberhalb oder in einem oberen Bereich des Sichtfensters, angeordnet sein. Insbesondere kann die Zuleitung derart ausgestaltet sein, dass die zugeführte verdampfte Flüssigkeit im Wesentlichen in einen Bereich vor dem Sichtfenster innerhalb des Behandlungsraums geleitet wird. Vorteilhafterweise kann ein Anschluss der Zuleitung an der Dichtmanschette und/oder in einem Bereich der Tür es ermöglichen, dass die verdampfte Flüssigkeit direkt an dem Sichtfenster in den Behandlungsraum eingelassen wird. Dadurch kann die verdampfte Flüssigkeit besser sichtbar sein, bzw. es ist möglich, eine ausreichende Sichtbarkeit mit einer geringeren Menge an verdampfter Flüssigkeit zu erreichen.

Gemäß einer weiteren bevorzugten Ausführungsform ist es vorgesehen, dass die Zuleitung an deren Mündung zumindest eine Düse umfasst. Die Düse kann einer Beschleunigung der verdampften Flüssigkeit und/oder einer gleichmäßigen Verteilung der verdampften Flüssigkeit, insbesondere vor dem Sichtfenster der Tür des Wäschepflegegeräts, dienen. Vorteilhafterweise kann durch die Düse, eine Sichtbarkeit der verdampften Flüssigkeit verbessert sein. Mit anderen Worten kann die versumpfte Flüssigkeit mit Hilfe der Düse rasch so in dem Behandlungsraum verteilt werden, dass der Nutzer die verdampfte Flüssigkeit wahrnehmen kann

Gemäß einem weiteren Aspekt der vorliegenden Erfindung wird Verfahren zum Verdampfen einer Flüssigkeit in einem Wäschepflegegerät bereitgestellt, umfassend die Schritte:
- Erzeugen eines Gases, insbesondere mit einer erfindungsgemäßen Verdampfungsvorrichtung wie hierin beschrieben,
- Starten eines Wäschebehandlungsprogramms, insbesondere eines Ozonbehandlungsprogramms und/oder eines Heißluftprogramms, in einem Behandlungsraum des Wäschepflegegeräts, und
- Zuführen des Gases in den Behandlungsraum, zumindest in der Anfangsphase des Wäschebehandlungsprogramms.

Die Merkmale und Vorteile, die für die Verdampfungsvorrichtung und das Wäschepflegegerät beschrieben worden sind, gelten in entsprechender Weise analog für das Verfahren und andersrum. Das Gas kann insbesondere eine verdampfte Flüssigkeit, bevorzugt verdampft durch die Verdampfungsvorrichtung, sein. Der Begriff Gas kann beispielsweise auch ein Flüssigkeit-Gas-Gemisch, insbesondere ein nebelartiges Gemisch bzw. Gas, umfassen. Besonders bevorzugt kann das Zuführen des Gases in der Anfangsphase, insbesondere nur in der Anfangsphase, des Wäschebehandlungsprogramms erfolgen. Es hat sich gezeigt, dass Nutzer das Wäschebehandlungsprogramm besonders in der Anfangsphase beobachten. Das Zuführen des Gases in der Anfangsphase kann daher besonders vorteilhaft sein. Andererseits kann ein Beschränken des Zuführens auf die Anfangsphase den Vorteil haben, dass Gas bzw. zu verdampfende Flüssigkeit eingespart werden kann. Die Anfangsphase kann vorzugsweise die Dauer vom Start des Wäschebehandlungsprogramms bis hin zu einer verstrichenen Zeit von 5 bis 180 Sekunden, bevorzugt 10 bis 120 Sekunden, besonders bevorzugt 20 bis 60 Sekunden, ganz besonders bevorzugt ungefähr 30 Sekunden bezeichnen. Es hat sich gezeigt, dass dieser Zeitraum derjenige Zeitraum ist, in dem Nutzer am ehesten dazu neigen, das Wäschebehandlungsprogramm zu beobachten. Ein Zeitraum von 20 bis 60 Sekunden, insbesondere ungefähr 30 Sekunden, kann dabei vorteilhafterweise eine ausreichend lange Zeitphase darstellen, um einen Benutzer hinsichtlich der ordnungsgemäßen Funktion des Wäschebehandlungsprogramms zu versichern und gleichzeitig ein Einsparen von nicht unbedingt zu diesem Zweck benötigter Flüssigkeit ermöglichen, gerade unter der Voraussetzung, dass das Gas ggf. noch eine Weile nach dem Einführen sichtbar sein kann.

Weitere Vorteile und Merkmale ergeben sich aus der nachfolgenden Beschreibung bevorzugter Ausführungsformen des erfindungsgemäßen Gegenstands mit Bezug auf die beigefügten Figuren. Die folgende Beschreibung dient lediglich der Verdeutlichung der Erfindung und sollte nicht derart aufgefasst werden, dass durch sie die beiliegenden Ansprüche auf eine der Ausführungsformen beschränkt werden. Einzelne Merkmale der einzelnen Ausführungsform können dabei im Rahmen der Erfindung miteinander kombiniert werden. Es zeigen:
- **Fig. 1**: eine schematische Seitenansicht einer Verdampfungsvorrichtung gemäß einer Ausführungsform der Erfindung,
- **Fig. 2**: eine schematische Seitenansicht auf einen Schnitt durch eine Verdampfungsvorrichtung gemäß einer Ausführungsform der Erfindung,
- **Fig. 3**: eine schematische Frontansicht auf ein Wäschepflegegerät gemäß einer Ausführungsform der Erfindung,
- **Fig. 4**: eine schematische Perspektivansicht einer Verbindung der Verdampfungsvorrichtung an einen Behandlungsraum des Wäschepflegegeräts gemäß einer Ausführungsform der Erfindung, und
- **Fig. 5**: ein Ablaufdiagramm eines Verfahrens gemäß einer Ausführungsform der Erfindung.

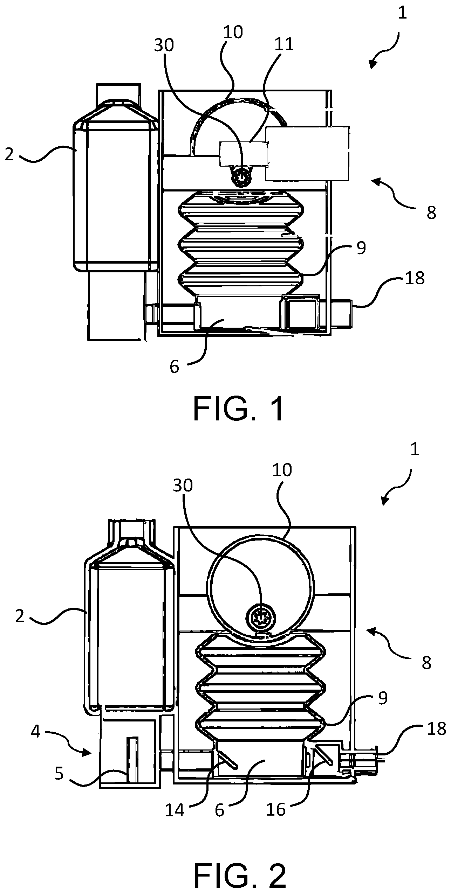

**Figur 1** ist eine schematische Seitenansicht einer Verdampfungsvorrichtung 1 gemäß einer Ausführungsform der Erfindung. Diese Ausführungsform der Verdampfungsvorrichtung 1 ist zudem in **Figur 2** in einer Schnittansicht aus der gleichen Perspektive gezeigt. Die Verdampfungsvorrichtung 1 umfasst einen mit einer Flüssigkeit gefüllten Flüssigkeitstank 2, einen Pumpenmechanismus 8 und einen Sammelbehälter 6. In dieser Ausführungsform ist, wie in der Schnittansicht der Figur 2 zu sehen ist, in einem unteren Bereich des Flüssigkeitstanks 2 ein Verdampfer 4 angeordnet, welcher ein Heizelement 5 in Form eines Heizstabs umfasst. Die Anordnung des Verdampfers 4 in dem unteren Bereich des Flüssigkeitstanks 2 gewährleistet, dass auch bei abnehmendem Füllstand des Flüssigkeitstanks 2 noch Flüssigkeit mit dem Heizelement 5 des Verdampfers 4 in Kontakt kommen kann. Der untere Bereich des Flüssigkeitstanks 2 und damit der Abschnitt des Flüssigkeitstanks 1 im Bereich der Position des Verdampfers 4 bzw. des Heizelements 5 ist an den Sammelbehälter 6 angeschlossen. Im Betrieb wird die Flüssigkeit im Flüssigkeitstank 1 durch das Heizelement 5 verdampft und durch den Pumpenmechanismus 8 in den Sammelbehälter 6 gesaugt. Der Pumpenmechanismus 8 umfasst dabei einen Blasebalg 9, der an den Sammelbehälter 6 von oben angeschlossen ist. Der Blasebalg wird durch ein Antriebsrad 10 mit exzentrischer Drehachse 30 angetrieben, wobei das Antriebsrad 10 wiederum durch ein Schneckengetriebe 11 angetrieben wird. Durch die exzentrischer Drehachse 30 des Antriebsrads 10 wird der Blasebalg 9 zyklisch gestreckt und zusammengestaucht, wenn das Antriebsrad 10 durch das Schneckengetriebe 11 angetrieben wird. Dabei wird durch ein Strecken des Blasebalgs 9 ein Unterdruck in dem Sammelbehälter 6 erzeugt, wodurch die verdampfte Flüssigkeit aus dem Flüssigkeitstank 1 in den Sammelbehälter 6 gesaugt wird. Um ein Ansaugen, z.B. von Gas oder Flüssigkeit, aus der zu einem Behandlungsraum 12 (hier nicht gezeigt) führenden Zuleitung 18 zu vermeiden, ist in dieser Ansaugphase ein Ausgangsventil 16 geschlossen. In einer darauffolgenden Pumpenphase, in der der Blasebalg 9 gestaucht wird, wird dieses Ausgangsventil 16 geöffnet, um ein zuleiten der verdampften Flüssigkeit über die Zuleitung 18 in den Behandlungsraum 12 zu ermöglichen. Gleichzeitig ist in dieser Pumpenhase ein Eingangsventil 14 zwischen Flüssigkeitstank 2 und Sammelbehälter 6 geschlossen, um ein Zurückpumpen der verdampften Flüssigkeit in den Flüssigkeitstank 2 zu verhindern.

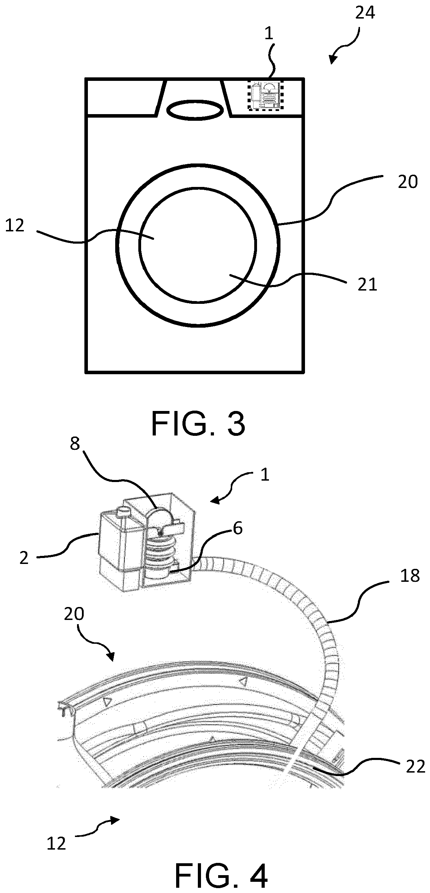

In **Figur 3** ist eine schematische Frontansicht auf ein Wäschepflegegerät 24 gemäß einer Ausführungsform der Erfindung in einer Betriebsstellung gezeigt. Die Betriebsstellung ist dabei so definiert, dass eine Unterseite der Fig. 3 einem Boden zugeordnet ist, auf dem das Wäschepflegegerät 24 steht. Dabei ist eine mögliche Position der Verdampfungsvorrichtung 1 in dem Wäschepflegegerät angezeigt, indem die Außenhülle des Wäschepflegegeräts 24 in dem Bereich eines eingezeichneten gestrichelten Kastens durchsichtig dargestellt ist. Die Verdampfungsvorrichtung 1 ist vorzugsweise oberhalb eines Behandlungsraums 12 des Wäschepflegegeräts 24 angeordnet. Der Behandlungsraum 12 ist hier durch ein Sichtfenster 21 einer Tür 20 des Wäschepflegegeräts 24 für einen Nutzer einsehbar.

In **Figur 4** ist dabei die Verbindung der Verdampfungsvorrichtung 1 an den Behandlungsraum 12 gemäß einer Ausführungsform der Erfindung in einer Perspektivansicht gezeigt. Dabei führt eine Zuleitung 18 von dem Sammelbehälter 6 der Verdampfungsvorrichtung zu einer Dichtmanschette 22 der Tür 20 des Wäschepflegegeräts 24. Über eine Öffnung in der Dichtmanschette 22 gelangt die verdampfte Flüssigkeit von der Zuleitung 18 in den Behandlungsraum 12 des Wäschepflegegeräts. Die Zuleitung 18 kann zum Beispiel ein Fluidschlauch sein. Gemäß einer Ausführungsform kann an der Anbindung der Zuleitung 18 an die Dichtmanschette 22 eine Düse vorgesehen sein, welche der Beschleunigung und/oder der gleichmäßigen Verteilung der verdampften Flüssigkeit im Bereich des Sichtfensters 21 des Behandlungsraums 12 dienen kann. Die Zuleitung des verdampften Fluids erfolgt demnach in unmittelbarer Nähe zu der Türe 20 und somit zu dem Sichtfenster 21. Somit kann bereits mit wenig verdampfter Flüssigkeit ein visueller Effekt in dem Behandlungsraum 12 erzeugt werden.

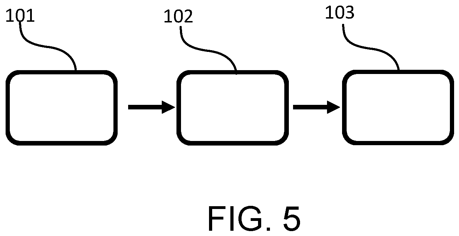

**Figur 5** zeigt ein Ablaufdiagramm eines Verfahrens zum Verdampfen einer Flüssigkeit in einem Wäschepflegegerät gemäß einer Ausführungsform der Erfindung. Dabei wird in einem ersten Verfahrensschritt 101 ein Gas erzeugt, wobei dazu bevorzugt eine erfindungsgemäße Verdampfungsvorrichtung 1 verwendet wird. In einem weiteren Schritt 102 wird ein Wäschebehandlungsprogramms zur Behandlung von Wäsche in einem Behandlungsraum 12 gestartet, wobei es sich insbesondere um ein Ozonbehandlungsprogramm und/oder ein Heißluftprogramm bzw. generell um ein Wäschebehandlungsprogramm, bei dem dem Behandlungsraum 12 keine für einen Nutzer sichtbaren Fluide zugeführt werden, handeln kann. Insbesondere in der Anfangsphase des Wäschebehandlungsprogramms wird dabei in einem weiteren Verfahrensschritt 103 das in Schritt 101 erzeugte Gas dem Behandlungsraum 12 zugeführt, wodurch vorteilhafterweise das Wäschebehandlungsprogramm für einen Nutzer optisch (besser) sichtbar werden kann. Damit erhält der Nutzer ein visuelles Feedback über den Ablauf eines Wäschebehandlungsprogramm.

### Bezugszeichenliste:

- 1: Verdampfungsvorrichtung
- 2: Flüssigkeitstank
- 4: Verdampfer
- 5: Heizelement
- 6: Sammelbehälter
- 8: Pumpenmechanismus
- 9: Blasebalg
- 10: Antriebsrad
- 11: Schneckengetriebe
- 12: Behandlungsraum
- 14: Eingangsventil
- 16: Ausgangsventil
- 18: Zuleitung
- 20: Tür
- 22: Dichtmanschette
- 30: Drehachse

- 101-105: Verfahrensschritte

## Patentansprüche

1. Verdampfungsvorrichtung (1) für ein Wäschepflegegerät (24), umfassend
einen mit einer Flüssigkeit befüllten und/oder befüllbaren Flüssigkeitstank (2),
einen Verdampfer (4), der dazu ausgestaltet ist, die Flüssigkeit zu verdampfen,
einen Sammelbehälter (6), der dazu ausgestaltet ist, die verdampfte Flüssigkeit aufzunehmen, und
einen Pumpenmechanismus (8), der dazu ausgestaltet ist, die verdampfte Flüssigkeit aus dem Sammelbehälter (6) einem Behandlungsraum (12) des Wäschepflegegeräts (24) zuzuführen.

2. Verdampfungsvorrichtung (1) gemäß Anspruch 1,
wobei der Pumpenmechanismus (8) einen Blasebalg (9) umfasst,
wobei der Pumpenmechanismus (8) zum Antreiben des Blasebalgs insbesondere ein Schneckengetriebe (11) und/oder ein Antriebsrad (10) mit exzentrischer Drehachse (30) umfasst.

3. Verdampfungsvorrichtung (1) gemäß einem der vorhergehenden Ansprüche,
wobei der Pumpenmechanismus (8) dazu ausgestaltet ist, in einer Ansaugphase durch Erzeugen von Unterdruck verdampfte Flüssigkeit aus dem Flüssigkeitstank (2) zu saugen und in einer Pumpphase durch Erzeugen von Überdruck die verdampfte Flüssigkeit dem Behandlungsraum (12) zuzuleiten.

4. Verdampfungsvorrichtung (1) gemäß Anspruch 3,
wobei in einer Verbindung zwischen dem Sammelbehälter (6) und dem Verdampfer (4) ein Eingangsventil (14) angeordnet ist, das dazu ausgestaltet ist, während der Ansaugphase offen und während der Pumpphase geschlossen zu sein und/oder
wobei in einer Verbindung zwischen dem Sammelbehälter (6) und dem Behandlungsraum (12) ein Ausgangsventil (16) angeordnet ist, das dazu ausgestaltet ist, während der Pumpphase offen und während der Ansaugphase geschlossen zu sein.

5. Verdampfungsvorrichtung (1) gemäß einem der vorhergehenden Ansprüche,
wobei die Flüssigkeit in dem Flüssigkeitstank (2) ein Desinfektionsmittel umfasst und/oder
wobei die Verdampfungsvorrichtung (1) einen Desinfektionsmittelbehälter und eine Desinfektionsmittelzuführleitung umfasst, und
wobei die Verdampfungsvorrichtung (1) dazu ausgestaltet ist, über die Desinfektionsmittelzuführleitung der verdampften Flüssigkeit Desinfektionsmittel zuzuführen.

6. Verdampfungsvorrichtung (1) gemäß einem der vorhergehenden Ansprüche,
wobei der Verdampfer (4) ein, insbesondere elektrisch betriebenes, Heizelement (5) umfasst.

7. Wäschepflegegerät (24) umfassend
eine Verdampfungsvorrichtung (1) zum Verdampfen von Flüssigkeit, insbesondere eine Verdampfungsvorrichtung (1) gemäß einem der Ansprüche 1 bis 6 und
einen Behandlungsraum (12) mit einer Fluidzufuhr, die dazu ausgestaltet ist, dem Behandlungsraum (12) ein Fluid zuzuführen, wobei der Behandlungsraum (12) mit der Verdampfungsvorrichtung (1) über eine Zuleitung (18) in Kommunikation steht,
eine Steuereinheit, die dazu ausgestaltet ist, dem Behandlungsraum (12), insbesondere während einer Ozonbehandlung und/oder einer Heißluftbehandlung, verdampfte Flüssigkeit zuzuführen.

8. Wäschepflegegerät (24) gemäß Anspruch 7,
wobei das Wäschepflegegerät (24) eine mit einer Tür (20) verschließbare Öffnung aufweist,
wobei an der Öffnung eine Dichtmanschette (22) angeordnet ist,
wobei die Zuleitung (18) an der Dichtmanschette (22) und/oder in einem Bereich der Tür an dem Behandlungsraum (12) angeschlossen ist.

9. Wäschepflegegerät (24) gemäß einem der Ansprüche 7 bis 8,
wobei die Zuleitung (18) an deren Mündung zumindest eine Düse umfasst.

10. Verfahren zum Verdampfen einer Flüssigkeit in einem Wäschepflegegerät (24), umfassend die Schritte:
- Erzeugen eines Gases, insbesondere mit einer Verdampfungsvorrichtung (1) gemäß einem der Ansprüche 1 bis 6,
- Starten eines Wäschebehandlungsprogramms, insbesondere eines Ozonbehandlungsprogramms und/oder eines Heißluftprogramms, in einem Behandlungsraum (12) des Wäschepflegegeräts (24), und
- Zuführen des Gases in den Behandlungsraum (12), zumindest in der Anfangsphase des Wäschebehandlungsprogramms.
